# EUROPEAN PATENT APPLICATION

(11) **EP 1 602 328 A1**
(43) Date of publication of application: **07.12.2005**
(21) Application number: 05104687.8
(22) Date of filing: 31.05.2005
(51) Int. Cl.: A61B 5/15

(54) **Flashback Blood Collection Needle**

(30) Priority: 02.06.2004 US 576217
(71) Applicant: Becton, Dickinson and Company, Franklin Lakes, New Jersey 07417-1880 (US)
(72) Inventor: Nair, Hareesh, Weehawken, New Jersey 07086 (US); Iskra, Michael, Bridgewater, NJ 08807 (US); Ellis, Robert, NJ 07430 (US); Bennett, Michael, Summit, NJ 07901 (US); Marsden, Stewart, NJ 07005 (US); Swenson, Kirk D., North Caldwell, NJ 07006 (US); Conway, Hugh T., Verona, NJ 07044 (US); Newby, C. Mark, Tuxedo, New Jersey 10987 (US); Bloch, Curtis, Sandy, UT 84070 (US); Levy, Richard, Sumter, SC 29150 (US); Wilkinson, Bradley, North Haledon, New Jersey 07508 (US); Schneider, James C., Wayne, NJ 07470 (US); Towns, Bryan, Union Bridge, MD Maryland 21791 (US)
(74) Representative: Weber, Thomas

(57) **Abstract**

The application refers to a self-venting blood collection needle assembly (10) for the extraction of at least one fluid sample into an evacuated container for laboratory testing, this blood collection needle assembly providing a clear or translucent flashback chamber (302) for blood to flow into, for visualization by the user to confirm successful vein entry. The self-venting mechanism (303) permits escape of air during use, and which, typically, also prevents an outflow of fluid, such as blood. Thus, air under venous pressure will he allowed to escape from the blood collection needle assembly until blood reaches the venting mechanism.

## Description

This application claims priority to U.S. Provisional Patent. Application Serial No. 60/576,217, which was filed on June 2, 2004.

### BACKGROUND OF THE INVENTION

### 1. Field of the Invention

The present invention relates to a device for collecting blood samples by performing venipuncture on a patient More particularly, the present invention relates to a needle assembly for multiple sample blood collection that allows a phlebotomist to determine whether vein entry has occurred, via a self-venting feature, when collecting a blood sample from a patient into an evacuated blood collection tube.

### 2. Description of Related Art

Venipuncture is the primary method used for acquiring blood samples for laboratory testing In performing venipuncture procedures, a phlebotomist must follow several steps simultaneously. Such steps include assessing the patient's overall physical and psychological condition so as to properly select a venipuncture site and technique The phlebotomist must also select the proper corresponding equipment perform the technique so as to control bleeding, and properly collect and identify fluid specimens for testing the phlebotomist must ascertain all of these coinciding factors, as such factors may adversely affect the distension of the vein and the length of the venipuncture procedure.

Various venipuncture devices have been developed which address the above-described problems. These devices incorporate a needle assembly having a hub defining a chamber therewithin, wherein a single cannula pointed at both ends, is affixed to the hub. The intravenous end of the cannula is adapted for penetration of a patient's vein, and the non-patient end of the cannula has a sealable sleeve and adapted for penetration of a penetrable stop positioned within an evacuated container

Upon vein entry with the intravenous end of the cannula, blood will flow though the cannula, into the sealable sleeve and into the hub chamber, which is clear or translucent for visualization ("flashback") Once air is vented from the hub chamber, the blood therein is pressurized each time the sealable sleeve is pushed toward the hub chamber upon activation of an evacuated container.

Due to the length of time between vein entry and flashback, the phlebotomist may erroneously believe that satisfactory vein entry has not been achieved since there is no immediate indication of vein entry in the see-through chamber. The phlebotomist may therefore unnecessarily repeat the venipuncture procedure, requiring replacement of the evacuated container and/or the needle assembly itself. Such a repetitive process prolongs the physical and emotional discomfort endured by the patient.

When flashback docs occur, the air in the needle hub is pressurized at venous pressure as the air in the injection cannula and flashback chamber is compressed into it. This pressurized air has nowhere to vent, therefore when the injection needle is withdrawn from the patient after blood collection procedure is completed, the pressurized air can force blood towards the injection needle tip.

It is therefore desirable to provide a fast, accurate and cost effective solution to conventional blood collection procedures upon which the phlebotomist may consistently rely on flashback to provide satisfactory venous entry. Moreover, it is particularly desirable to provide a blood collection needle assembly that permits blood flow through a relatively short needle directly into a flashback chamber and allows the venting of pressurized air from within the needle assembly, thereby providing immediate indication of successful vein entry and preventing blood leakage from the assembly. As used herein, venting media indicates the actual element that vents the air, e.g., plug, coating, finish, etc).

### SUMMARY OF THE INVENTION

The invention is a self-venting blood col lection needle assembly for the extraction of at least one fluid sample into an evacuated container for laboratory testing, this blood collection needle assembly providing a clear or translucent flashback chamber for blood to flow into, for visualization by the user to confirm successful vein entry. The self-venting mechanism permits escape of air during use, and which, typically, also prevents an outflow of fluid, such as blood. As used herein, venting mechanism indicates one or more features or elements that provide venting of air, but which, typically, prevent fluid front passing through thus, air under venous pressure will be allowed to escape from the blood collection needle assembly until blood reaches the venting mechanism. The venting mechanism then will seal, or prevent blood flow around or through it, to allow blood to be collected into evacuated collection tubes or into other appropriate blood collection receptacles the invention thus provides good flash visualization within the flashback chamber, without affecting accepted blood collection processes. A variety of venting mechanisms, venting media and venting locations arc suitable, as set forth below

A self-venting blood collection needle assembly is provided for collecting at least one fluid sample from a patient for subsequent discharge into at least one evacuated container The self-venting blood collection needle assembly of the present invention includes a hub having a fluid inlet end defined by a cylindrical exterior wall. The wall delineates a flashback chamber within the hub for retention of a blood sample therein and at least a portion of the walls transparent or translucent to provide for external visualization of the flash. The hub further includes a fluid outlet end in communication with said fluid inlet end. A first inlet cannula in fluid communication with the blood inlet end extends outwardly therefrom. The first cannula has an interior proximal extremity positioned proximate the chamber and an exterior distal extremity opposed thereto that is adapted for puncture of a patient's vein. Similarly, a second non-patient outlet cannula is provided in fluid communication with the fluid outlet end and extends outwardly therefrom. The second non-patient cannula has an interior distal extremity positioned proximate the first interior extremity and further includes an exterior proximal extremity opposed to said second interior extremity. The second exterior proximal extremity is adapted for puncture of a penetrable stopper in an evacuated container The second non-patient cannula further includes a scalable multiple sample sleeve. However a single cannula, which contains a notch in it, can also be used instead of the two cannula design. Another alternative cannula design uses a single cannula with no notch such that flashback occurs after flow through the non-patient cannula proximal end and around the multiple sample sleeve and then through the vent. External portions of the hub near the proximal end thereof may be formed with an array of external threads or other mounting structure to enable the blood collection needle assembly to be mounted to a collection tube holder or other such medical device. Or, the holder may be pre-attached with the needle assembly.

In one embodiment, the venting mechanism is located in the hub within the flashback chamber. The use of the 2 cannula design or the notched single cannula is appropriate with this configuration. The venting mechanism thereby provides communication between the fluid passage and the surrounding environment through the hub.

In another embodiment, the venting mechanism is located beyond the non-patient cannula proximal end, which means that the air passes through the non-patient cannula proximal end from which blood is drawn, and then through the vent. A single cannula with no notch is appropriate here, however all cannula configurations can be used. Specifically, air is vented from the fluid passage and out of the non-patient cannula proximal end where it further flows through the space between needle exterior and multiple sample sleeve. The air then flows through the venting mechanism, which may be at the non-patient barb, the non-patient hub thicad, the non-patient hub body, the multiple sample sleeve, or other location or combination of locations that are beyond the non-patient cannula proximal end. The collection tube, which is applied at the non-patient cannula proximal end, draws blood from only the fluid passage and not from the vent space. This embodiment thus enables blood to flow through the entire collection path. It also avoids the blood specimen coming in contact with the vent, which could potentially cause platelet activation, contamination or other undesirable result. It also avoids air being sucked back into the fluid passage when the evacuated tube is applied. It should also be noted that this embodiment necessitates a smaller flash chamber when compared to the previous embodiment where the vent is located in the hub.

Another embodiment of this invention has a venting mechanism comprised of a unified hub that is at least partially constructed of porous material such as a sintered plastic, ceramic or metal. The porous material can be arranged to provide venting of air either before that air enters the non-patient cannula, or after the air flows through the non-patient cannula, out the proximal end, and into the space between the cannula and a multiple sample sleeve. The porous material provides venting of the air but blocks leakage of the blood. In the typical embodiment, the porous material is hydrophobic. The porous material may further contain materials that swell upon wetting to further contain the blood. Other venting methods are also possible The internal passage wall's surface may be coated with a sealant to prevent contamination of the blood sample by the porous material. This embodiment enables blood to flow through the entire collection path. Optionally, the hub can be permanently bonded to a tube holder obviating the need or inconvenience of threaded connections. Bonding to the holder may be accomplished by solvent, welding, heat, pressure or and other convenient means or combination thereof. Such an integrated device is highly efficient to manufacture, and promotes safe medical practice by having the holder be discarded with the needle.

In a further embodiment, the venting mechanism involves venting an through a side opening located in the hub to the exterior, where the opening is covered by a venting material having a shape, which mechanically holds the venting material in or on the opening. Preferably, the vent material is hydrophobic such that the surface tension also prevents leakage. The vent media material in this embodiment typically has an elastic property and shape such that spring energy holds the vent material onto the device. For example, it is possible to use a C-shuped vent in which distortion of the shape is required for the vent to stretch over the receiving structure on the hub. Once the vent is placed over the receiving structure, it is released and fully maintained in place using its own resiliency and in absence of bonding materials such as epoxies, which could be disadvantageously absorbed into the vent. The vent mechanism of this embodiment could alternatively involve first compressing a vent material, placing the material into the opening, and releasing the vent material to expand into the opening. This embodiment, enables efficient mass production

### DESCRIPTION OF THE DRAWINGS

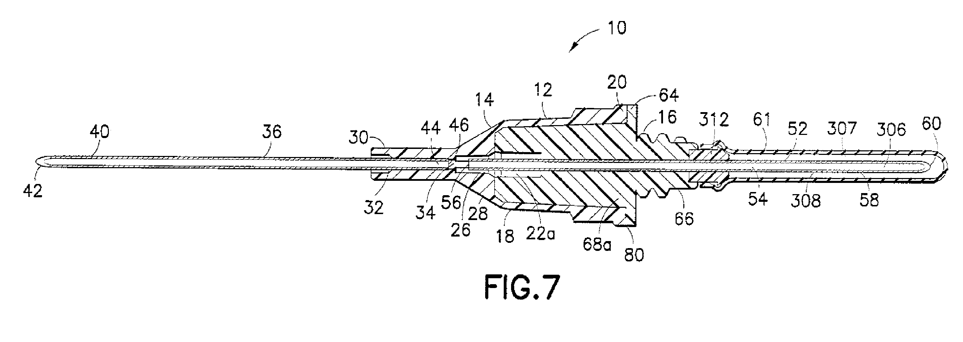

FIG. 1 is a perspective view of blood collection needle assembly.
FIG. 2 is a cross-sectional view of an alternate embodiment of the blood collection needle assembly of the present invention.
FIG. 3 is a cross-sectional view similar to FIG. 2, but showing an alternate embodiment of the invention.
FIG. 4A is a cross-sectional view similar to FIG. 2, but showing an alternate embodiment of the invention.
FIG. 48 is a side elevation view of FIG 4B

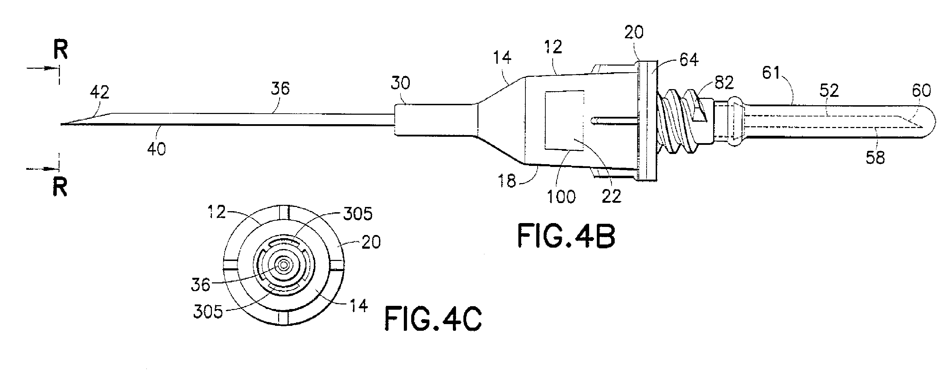

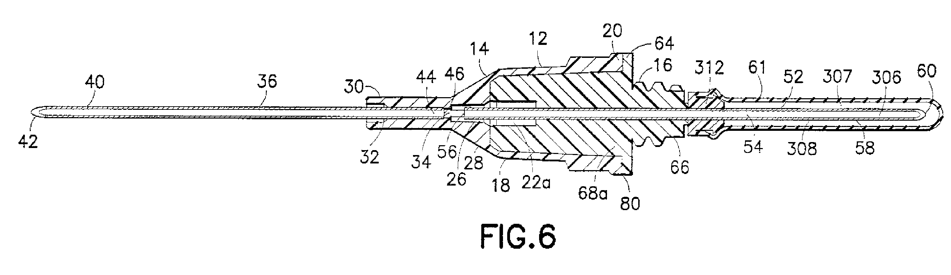

FIG. 4C is a side elevation view of FIG. 4BA from the aspect of R-R.

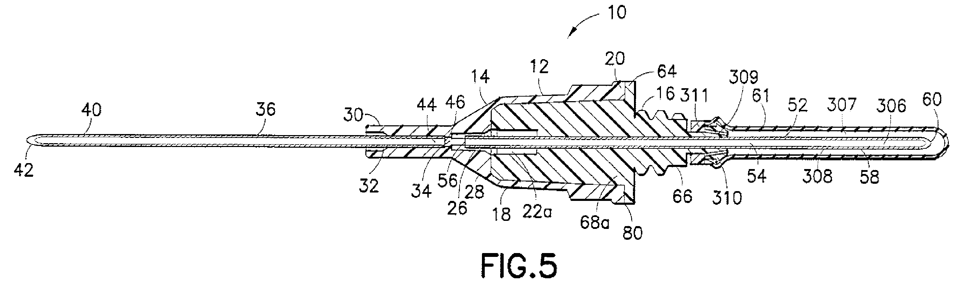

FIG. 5 is a cross-sectional side view of an embodiment of the blood collection needle assembly.
Fid. 6 is a cross-sectional view similar to FIG. 5, but showing an alternate embodiment of the invention
FIG. 7 is a cross-sectional view similar to FIG. 5, but showing an alternate embodiment of the invention.

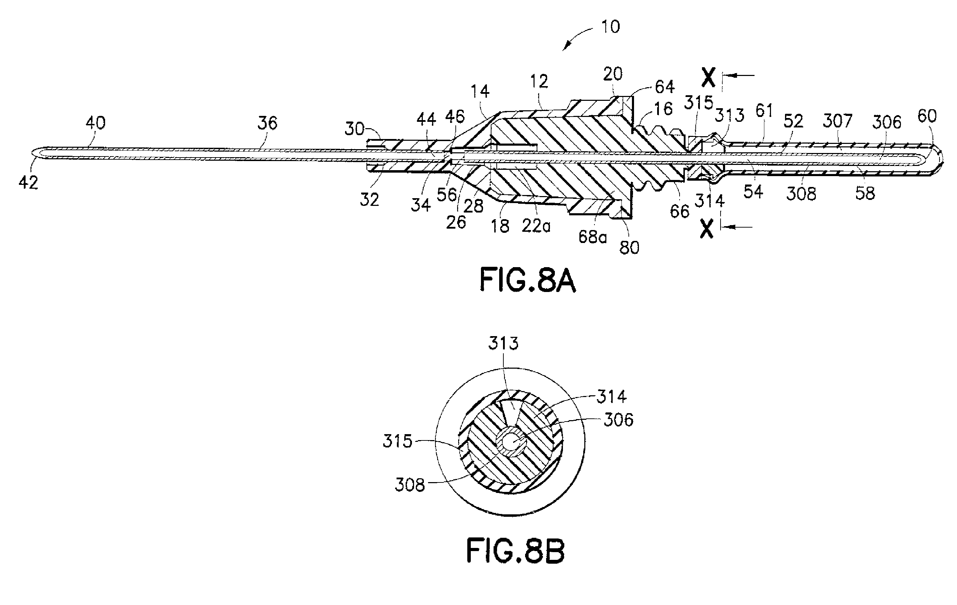

FIG. 8A is a cross-sectional view similar to FIG. 5, but showing an alternate embodiment of the invention.
FIG. 8B is a side elevation view of FIG. 8A from the aspect of X-X.

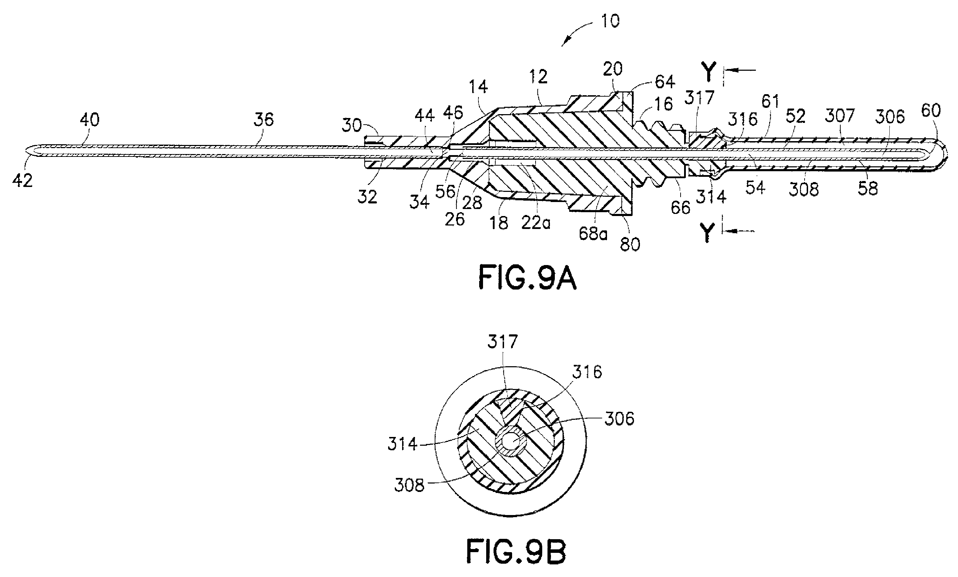

FIG. 9A is a cross-scctional view similar to FIG. 5, but showing an alternate embodiment of the invention.
FIG. 9B is a side elevation view of FIG. 9A from the aspect of Y-Y

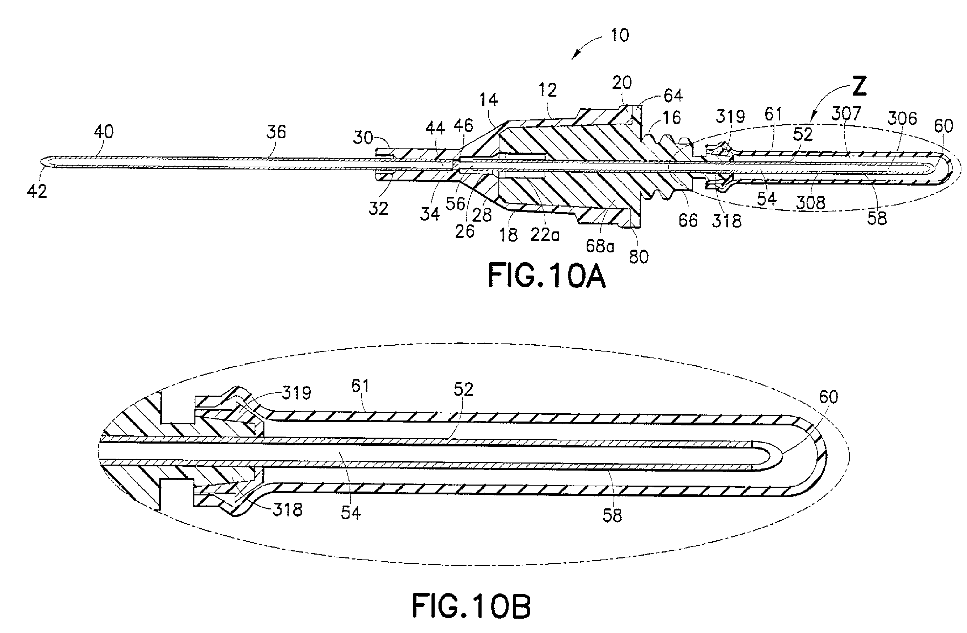

FIG. 10A is a cross-sectional view similar to FIG. 5, but showing an alternate embodiment of the invention
FIG. 10B is a magnified view of FIG. 10A from the aspect of Detail Z

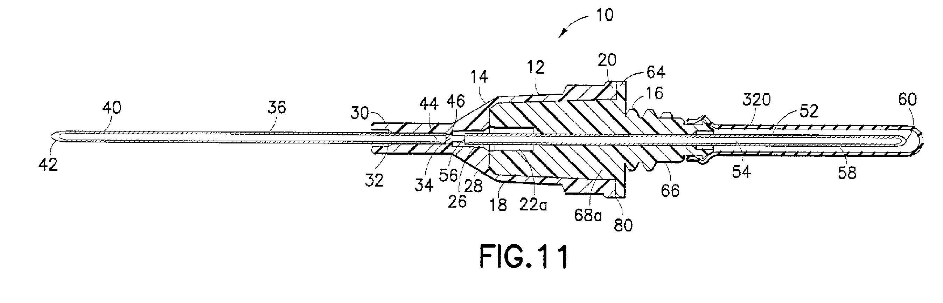

FIG. 11 is a cross-sectional side view of an embodiment of the blood collection needle assembly.

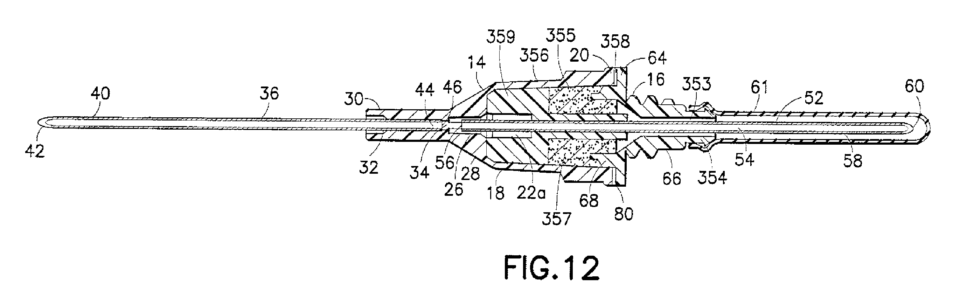

FIG. 12 is a cross-sectional side view or an embodiment of the blood collection needle assembly.

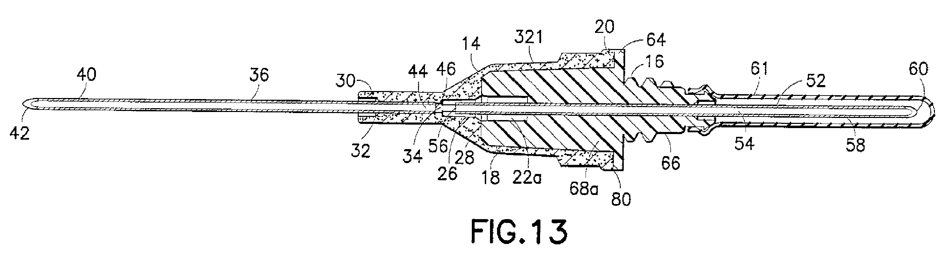

FIG. 13 is a cross-sectional side view of an embodiment of the blood collection needle assembly.

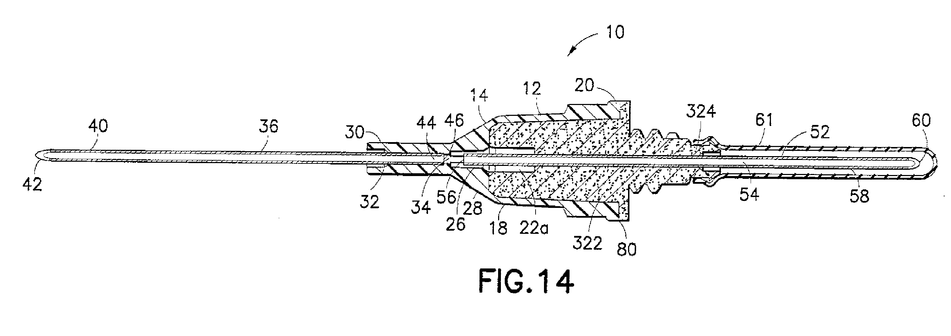

FIG. 14 is a cross-sectional side view of an embodiment of the blood collection needle assembly.

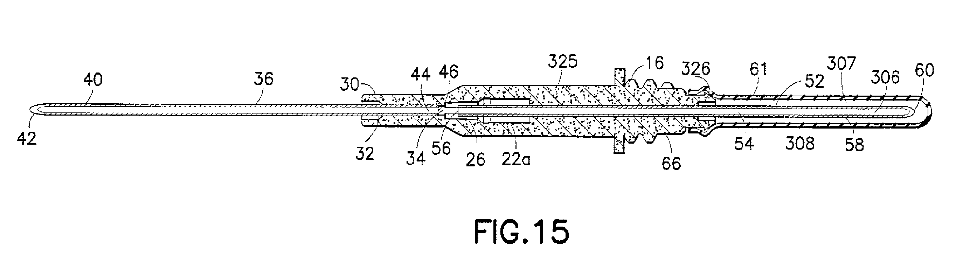

FIG 15 is a cross-sectional side view of an embodiment of the blood collection needle assembly.

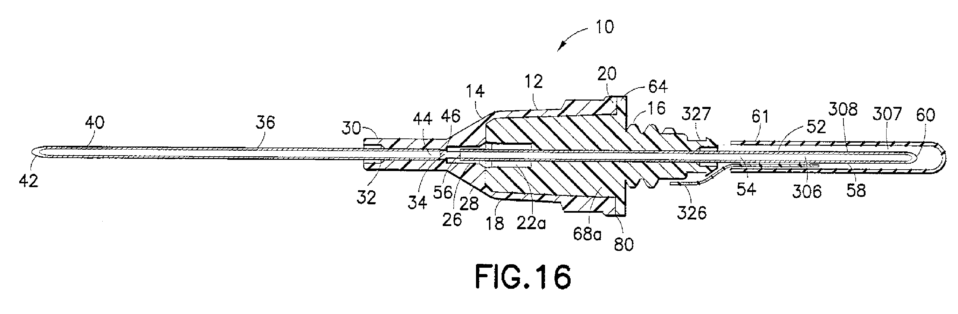

FIG. 16 is a cross-sectional side view of an embodiment of the blood collection needle assembly

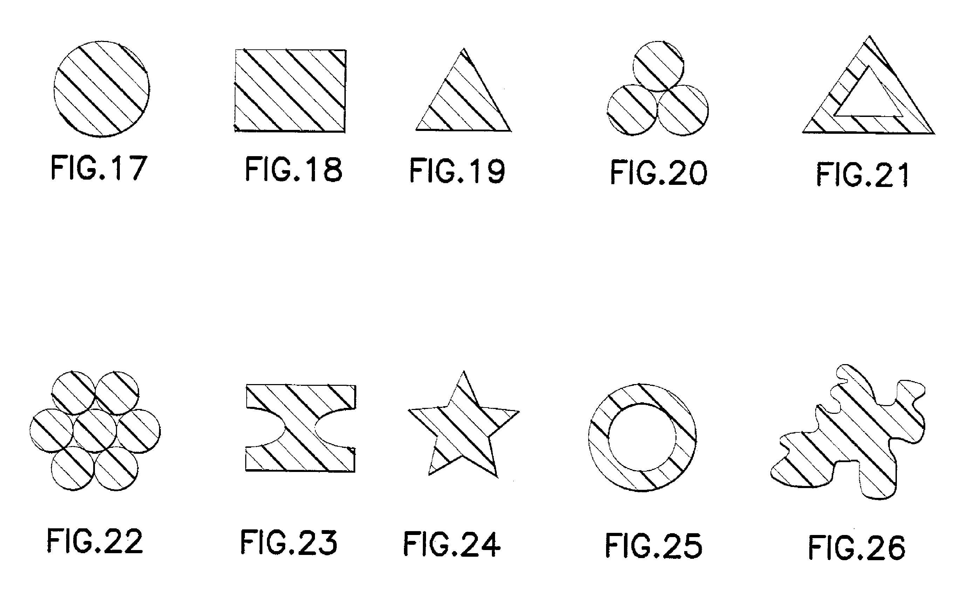

FIG. 17 is a cross-sectional view of an embodiment of the breathable cord design.
FIG. 18 is also a cross-sectional side view similar to FIG. 17, but showing an alternate embodiment of the invention
FIG. 19 is also a cross-sectional side view similar to FIG. 17, but showing an alternate embodiment of the invention.
FIG. 20 is also a cross-sectional side view similar to FIG. 17, but showing an alternate embodiment of the invention.
FIG. 21 is also a cross-sectional side view similar to FIG. 17, but showing an alternate embodiment of the invention.
FIG. 22 is also a cross-sectional side view similar to FIG. 17, but showing an alternate embodiment of the invention.
FIG. 23 is also a cross-sectional side view similar to FIG. 17, but showing an alternate embodiment of the invention.
FIG. 24 is also a cross-sectional side view similar to FIG. 17, but showing an alternate embodiment of the invention
FIG. 25 is also a cross-sectional side view similar to FIG. 17, but showing an alternate embodiment of the invention.
FIG 26 is also a cross-sectional side view similar to FIG. 17, but showing an alternate embodiment of the invention.

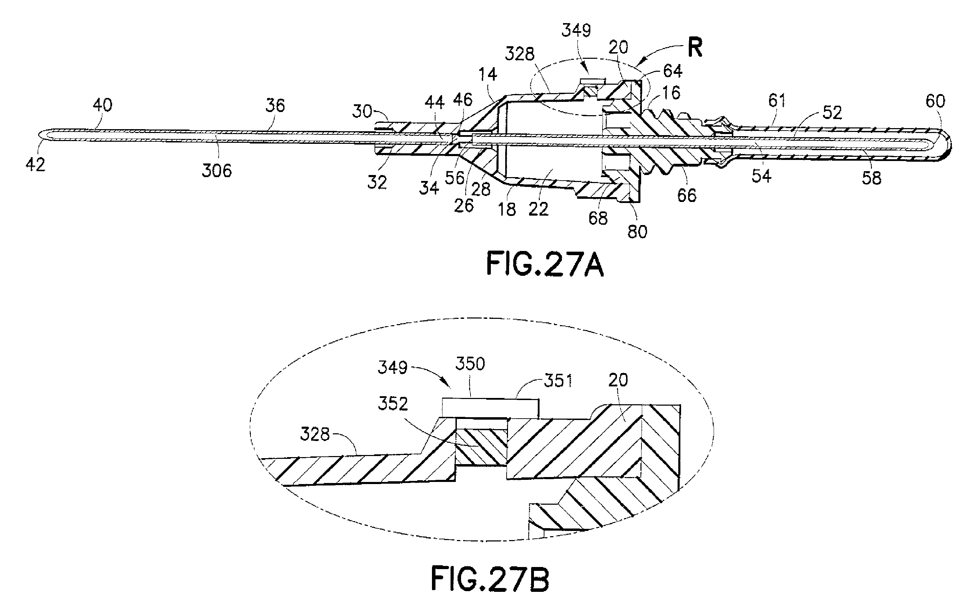

FIG. 27A is a cross-sectional side view of an embodiment of the blood collection needle assembly.
FIG. 27B is a magnified view of FIG. 27A from the aspect of Detail R.

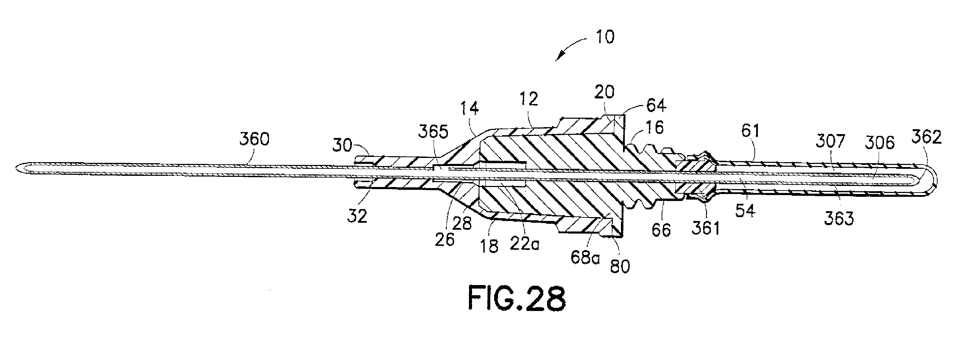

FIG. 28 is a cross-sectional side view of an embodiment of the blood collection needle assembly.

### DETAILED DESCRIPTION

The present invention provides a self-venting blood collection needle assembly with a self-venting mechanism that permits escape of air, while preventing an outflow of fluid such as blood, that provides a visual indication of vein entry ("flashback") upon collection of a blood or other fluid sample from a patient into one or more evacuated blood collection tubes

It should be noted that the vent media could be, for example, a distinct physical element such as a plug or insert, a integral portion of a device that has been treated such as by laser drilling or has been formed in whole or in part from a porous material, or a coating, layer, etc formed by disposing a material onto the device, e.g., by dipping, coating, spraying or the like.

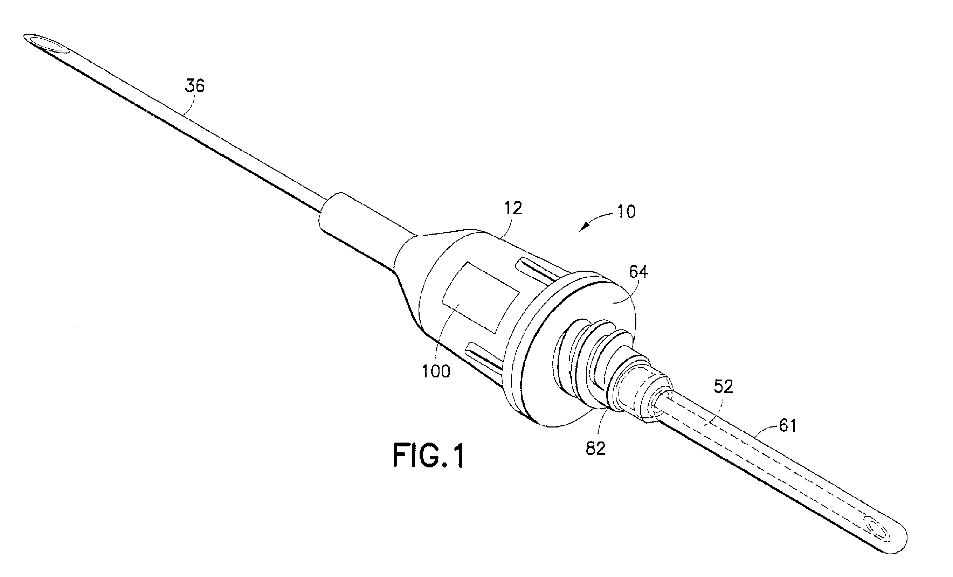

A blood collection needle assembly in accordance with an embodiment of the subject invention is identified in **FIG.** 1. A needle assembly **10** includes a hub **12,** which supports a fluid inlet needle (first cannula) **36** on one side and a fluid outlet non-patient needle (second cannula) **52** on an opposite side thereof. Fluid collected from the first cannula is immediately visualized in a flashback chamber in the hub **12,** through the hub, via a translucent window **100** to provide a timely indication of proper vein entry with the help of a venting mechanism.

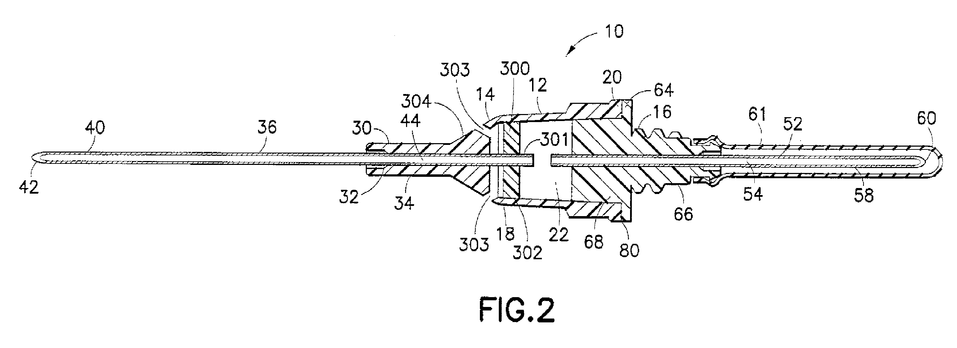

FIGs. 2-28 show various embodiments of the invention, including various configurations of venting mechanisms in blood collection needle assemblies. In particular, FIGs. 2, 3, 4A, 4B and 4C reflect embodiments in which a venting mechanism is located in the hub portion. FIGs. 5-12 reflect embodiments where the venting mechanism is located beyond the proximal end **60** of the non-patient cannula. FIGs. 13 and 14 reflect embodiments where part of the needle hub acts as the vent media. FIG. 15 reflects an embodiment where the venting mechanism is a unified hub **132.** FIGs. 16-26 reflect embodiments where the venting mechanism is a breathable cord. FIGs. 27A and 27B reflect embodiments where the venting mechanism is a one-way valve FIG. 28 reflects an embodiment, which contains one notched cannula. It should be noted that in each of the following embodiments where there are two cannula, one notched cannula could equally be used.

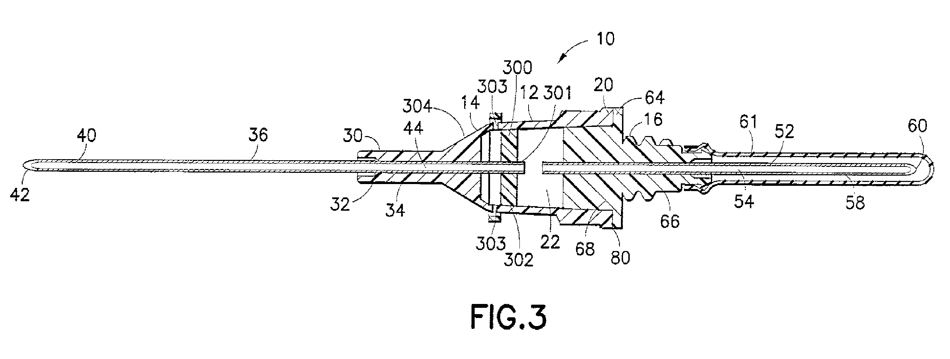

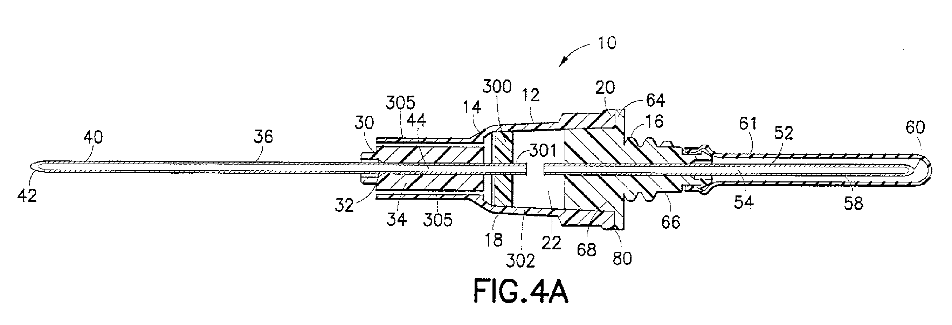

FIGs. 2, 3, 4A, 4B and 4C, show a venting mechanism in which a venting ring **300** is situated around the proximal end **301** of the first cannula **36.** The venting ring forms an interference fit with the inside wall of the flashback chamber **302**. Air can then escape by passing though the venting ring and out of venting apertures **303** to the ambient surroundings. The apertures may be located anywhere in the distal end of the hub. In FIGs. 2 and 3, the apertures are located in the distal end of the hub **304** An alternative embodiment can be send in FIGs. 4A, 4B and 4C in which the venting apertures are longitudinal venting windows **305** which abut the distal side of the venting ring **300.**

Several embodiments involve a venting mechanism location beyond the proximal end **60** of the non-patient cannula. This vent mechanism advantageously uses a smaller flashback chamber **22a** in order to reduce the amount of air to be vented from the hub assembly **12** when compared to the previous embodiments such as FIGs 2, 3, 4A, 4B and 4C. The following embodiments use a larger annular flange **68a** in order to produce a smaller flashback chamber **22a** however other design approaches can be used. Examples of these design approaches are a window of translucent/transparent material and/or a portion of the flashback chamber that is closer to the hub surface to enhance flash visualization. It should also be noted that the flashback chamber or window could also be located at the end of the air/blood path, especially if the embodiment uses a single cannula with no notch.

In the embodiment of FIG. 5, air flows from the fluid passage **306** and out of the non-patient cannula proximal end **60** where it further flows through the space **307** between needle exterior **308** and multiple sample sleeve **61** interior to the location of the vent **309,** which consists of a passage through the non-patient barb **310** then through the venting plug **311** that permits an outflow of air, but prevents an outflow of blood or other fluids to the ambient surroundings.

FIGs 6, 7, 8A, 8B, 9A, 9B, 10A, 10B, and demonstrate more embodiments where venting mechanisms are located beyond the proximal end **60** of the non-patient cannula. The embodiments in **FIGs.** 6 and 7 show a venting plug **312** that also functions as at least a portion of the non-patient barb. In both embodiments, as shown in the Figures, at least a portion of the vent has access to the exterior, to vent air passing through **FIGs.** 8A and 8B show an embodiment in which a slit **313** in the non-patient barb **314** allows air to escape to a venting disc **315** FIGs. 9A and 9B show an alternative embodiment in which a slit **316** in the non-patient barb **314** contains a venting plug **317** FIGs. 10A and 10B show a modified non-patient barb design **318** in which a venting sleeve **319** allows air to escape. In each embodiment the vent mechanism (and like elements described herein) permits an outflow of air, but prevents an outflow of blood or other fluids to the ambient surroundings.

FIG 11 shows another embodiment of the venting mechanism location beyond the proximal end **60** of the non-patient cannula. According to this embodiment, air is vented through the material of the multiple sample sleeve **320** itself, which functions as the vent media that prevents a flow of fluid from non-patient cannula **52.** Multiple sample sleeve **320** maintains its normal function of being pierced by pointed proximal end **60** of non-patient cannula **52** in response to forces generated by a stopper on an evacuated collection tube. It is possible to make a multiple sample sleeve **320** that also functions as a vent by forming the sleeve from a porous hydrophobic material, such as those disclosed above.

FIG. 12 shows another embodiment in which air escapes through a small channel **353** inbetween the non-patient needle exterior **52** and the non-patient barb **354** and then through the base **64** and annular flange **68** into a reservoir **355** in the hub **356** that contains the vent media **357** and out to the surrounding atmosphere through a channel **358** at the interface **80** between the hub **356** and the base **64** of the non-patient hub. A hub insert **359** forms the reservoir **355.**

FIGs. 13 and 14 show a venting mechanism in which the hub **321** or the non-patient thread assembly **322,** are made from a porous material and thus act as the vent media. FIG 13 shows the hub **321** as the vent media, which allows air to escape from the annular trench **26** to the surrounding atmosphere through the taper **28** of the hub **321.** FIG 14 shows the non-patient thread assembly **322** as the vent media. This embodiment allows air the escape through the walls of the flashback chamber **22a** and then through the non patient barb **324.**

FIG. 15 shows a venting mechanism, which is a unified one piece hub **325,** that is made form a porous material vent media that allows air to escape through the walls of the annular trench 26 and the flashback chamber **22a** and from the fluid passage **306** and out of the non-patient cannula proximal end **60** where it further flows through the space **307** between needle exterior **308** and multiple sample sleeve **61** interior, then through the non patient barb **326** to the surrounding atmosphere. And because of the nature of the unified hub **325,** the holder can be pre-attached by bonding, rather than by providing threads on the hub **325**. However threads can be provided if desired, as shown. Bonding of a tube holder to the unified non-patient hub **325** may be accomplished by solvent, welding, heat, pressure or and other convenient means or combination thereof.

FIG. 16 shows the venting mechanism of a breathable venting cord **326** located between the sealing surfaces of the multiple sample sleeve **61** interior and non patient barb **327.** The presence of the cord in the sealing surface allows air to escape from the space **307** between needle exterior **308** and multiple sample sleeve **61** interior but prevents leakage of a fluid through either the absorbent nature of the cord material and / or the very small size of the channel created by the cord. Venting using this mechanism may be accomplished by locating the vent media (e.g., placing, coating, or treating) between any one or multiple scaling surfaces along the fluid passage **306.** FIGs. 17 to 26 show cross-sections of suitable breathable cords. Other shapes, or combinations of such profiles, may also be used. Cords may be extruded or woven, for example and the application of a hydrophobic coating such as wax may be advantageous.

In a Further embodiment, the venting mechanism utilizes a one-way valve located somewhere along the fluid passage. The valve allows air to escape but shuts closed when vacuum is applied thus, when an evacuated collection tube is applied at the needle tip, the tube draws blood from the fluid passage but not air. FIGs. 27A and 27B show an example of a one-way valve. The venting mechanism may be at any location or locations along the fluid passage 306, but is typically at the hub **328.** The valve **349** itself may be a thin flap such as plastic film **350** covering the vent, a deformable seal such as a rubber or plastic duckbill valve, a deformable wrap over the vent, or any other means or combination of these The valve **349** may be proximal or distal with respect to the vent. In the embodiment shown in **FIG** 27B the thin plastic film valve **349** is attached to the hub **328** along one scaled edge of the film **351,** so that on the initial venous puncture, air is pushed out of the fluid passage **306** under venous pressure through the porous vent plug **352** and out from underneath the unsecured edges of the plastic film **349.** However when a vacuum is applied to the fluid passage **306** (via the attachment of a blood collection tube) the thin plastic film valve **349** is pulled tight against the porous vent plug **352** thereby scaling the vent and preventing air from reentering the fluid passage. This embodiment may thus provide a primary or back-up feature to prevent air from reentering the system after venting occurs.

FIG 28 shows a blood collection needle assembly that contains a single cannula **360** and a venting plug **361** that also functions as the non-patient barb. The cannula **360** is positioned in bore **34** such that a hole **365** in the cannula **360** lies at the location of the annular trench **26** so as to remain in fluid communication therewith. Once cannula **360** is properly positioned, it may be frictionally engaged by bore **34** or affixed therein by means of an adhesive or the like. On venous entry air flows from the fluid passage **306** and out of the cannula proximal end **362** where it further flows through the space **307** between needle exterior **363** and multiple sample sleeve **61** interior to the location of the venting plug **361**.

As will be apparent to one skilled in the art, it is possible to combine one or more vent mechanisms in a single device, or put identical vent mechanisms at more than one location in a device. Moreover, 1 is possible to use any of a variety of vent media in the vent mechanisms of the invention In addition, vent mechanisms herein may be applicable in a variety of devices other than blood collection needle assemblies.

Vent media, as used herein, can include, for example, either or a combination of:
- a porous plug formed from a matrix or carrier material, typically hydrophobic, that is coated with, impregnated with, or otherwise, contains a hydrophilic material that swells on contact with aqueous or water containing substances. This swellable nature thereby provides the sealing function in the vent upon contact with blood;
- an air vent provided through a matte finish, micro-sized channels, laser drilled holes, tortuous path, porous material or a vent provided between sealing surfaces, e.g., in a cord in which the holes, gaps or channels are large enough to permit airflow but small enough to prevent blood leakage;
- a porous plug that becomes sealed upon contact with blood using biological phenomena, e.g., by clotting and/or cell agglutination that blocks the vent;
- a superabsorbant material to seal the vent by swelling on contact with an aqueous fluid; or
- a one-way valve, e.g., a thin flap such as plastic film covering a vent, a deformable seal such as a rubber or plastic duckbill valve, or a deformable wrap over a vent.

Typically, a porous plug is formed from a hydrophobic material, such as high-density polyethylene (HDPE), which is coated with, impregnated with, or otherwise contains a hydrophilic material such as carboxymethylcellulose (CMC) or a polyacrylate Alternative hydrophobic materials include but are not limited to polytetrafluoroethylene (PTFE), ultra-high molecular weight polyethylene (UHMWPE), Nylon 6, polypropylene (PP), polyvinylidine fluoride (PVDF) or polyethersulfone (PES). Vent media according to the invention, as discussed in more detail below, may alternatively use a matte finish, one or more micro-sized channels, laser drilled holes, breathable cord, superabsorbant, one-way valve, or any other means or any combination of these, as appropriate and suitable for the venting mechanism and location. Thus, reference herein to any particular vent media, e.g., a plug, shall not be limiting, but shall be intended to include appropriate substitutes of other vent media.

An embodiment of the vent media consists of micro-sized holes formed in an exterior wall The holes are large enough to permit airflow but small enough to prevent blood leakage The vent holes may be any number including a single hole although multiple holes are typical for a more reliable function. The holes may be laser-drilled, meaning that they may be burned through the wall or substrate using one or more laser beams The substrate may be any convenient material although thin plastic or plastic film is typical. The vent mechanism may include a one-way valve as previously described. The vent mechanism may be located at any convenient space along the fluid passage in the injection or non-patient cannula, hub or in an added component although location at the proximal end is typical to provide flash along the full length of the tubing.

A porous plug that becomes scaled upon contact with blood using biological phenomena may use, for example, a porous material such as a sintered plastic, ceramic or metal, or a breathable cord, or by locating the biological agent in small holes or spaces between parts. The vent may be of any convenient shape the venting may be at any location or locations along the fluid passage, but is preferably at the proximal end such as at the hub near the collection device. The vent is typically made from, contains, is adjacent to, or works in collaboration with, a stimulant that interacts with blood to promote clotting and/or cell agglutination such that the clot and/or clumped cells block ongoing flow of blood through the vent. An example a clotting stimulant is silica or crushed glass, or fiberglass. An example of an agglutinizing agent is lectin An example of a platelet activator is collagen or thrombin A neutralizer for anti-coagulant such as protomine sulfate may be included. The biological stimulant may be applied using any convenient process including as a powder, a solution, a suspension, a slurry, or any other form. It may be dried of lyophilized

Various other changes and modifications may be effected therein by one skilled in the art without departing from the scope or spirit of the invention, and it is intended to claim all such changes and modifications as fall within the scope of the invention.

## Claims

1. A needle assembly comprising:
a hub having an inlet end, an outlet end and a chamber formed between said ends;
an inlet cannula having a distal end and a lumen extending therethrough, said inlet cannula being mounted to said hub such that said distal end of said inlet cannula is external of said hub and such that said lumen through said inlet cannula communicates with said chamber;
an outlet cannula having a proximal end and a lumen extending therethrough, said outlet cannula being mounted to said hub such that said proximal end of said outlet cannula is external of said hub and such that said hunen of said outlet cannula communicates with said chamber
wherein said inlet and outlet cannula are distinct or comprise a single cannula with an aperture providing communication with said chamber;
a multiple sample sleeve mounted over portions of said outlet cannula disposed externally of said hub, wherein said inlet cannula lumen, said chamber, said outlet cannula lumen and the internal surface of said multiple sample sleeve form a fluid passage; and
a venting mechanism providing communication between said fluid passage and ambient surroundings, wherein said venting mechanism permits an out flow of air from the needle assembly to said ambient surroundings through said venting mechanism, and wherein n said venting mechanism substantially prevents an outflow of fluid from said needle assembly to said ambient surroundings through said venting mechanism,

2. The needle assembly of Claim 1, wherein said venting mechanism extends through said hub.

3. The needle assembly of claim 2, wherein said venting mechanism comprises a vent media.

4. The needle assembly of Claim 2, wherein said venting mechanism comprises at least one aperture through said hub, said aperture containing a venting plug

5. the needle assembly of Claim 4, wherein said venting mechanism further comprises a one-way valve

6. The needle assembly or Claim I, wherein said venting mechanism comprises at least one aperture through said hub and a venting ring surrounding said inlet cannula, such that said venting ling is located proximally of said aperture through said hub within said chamber.

7. The needle assembly of Claim 1, wherein said venting mechanism is located in said passage beyond said proximal end of said outlet cannula.

8. The needle assembly of Claim 7, wherein said venting mechanism comprises a vent media.

9. The needle assembly of claim 1, wherein said hub further comprises a non-patient barb, and wherein said venting mechanism extends through said non-patient barb.

10. The needle assembly of Claim 9, wherein said venting mechanism comprises a vent media

11. The needle assembly of Claim 9, wherein said venting mechanism comprises a passage through said non patient barb and a venting plug located in said passage.

12. The needle assembly of claim 9, wherein said venting mechanism comprises a slit through said non patient barb and a venting disc.

13. The needle assembly of Claim 9, wherein said venting mechanism comprises at least one aperture through said non patient barb and a venting plug located in said aperture.

14. The needle assembly of Claim 1, wherein said hub further comprises a non-patient barb, and wherein at least a portion of said non-patient barb comprises said venting mechanism.

15. The needle assembly of Claim 14, wherein said venting mechanism comprises a vent media.

16. The needle assembly of Claim 14, wherein at least a portion of said non-patient barb is formed from a porous hydrophobic material or said portion of said non-patient barb comprises a porous material with a hydrophobic surface.

17. The needle assembly of Claim 14, wherein said venting mechanism comprises a porous material impregnated with a hydrophilic material.

18. The needle assembly of Claim 1, wherein said hub further composes a non-patient barb to which said multiple sample sleeve is securely mounted, and wherein said venting mechanism is located between said non-patient barb and said multiple sample sleeve.

19. The needle assembly of Claim 18, wherein said venting mechanism comprises a venting sleeve.

20. The needle assembly of Claim 19, wherein said venting sleeve comprises a porous hydrophobic material or said venting sleeve comprises a porous material with a hydrophobic surface

21. The needle assembly of Claim 18, wherein said venting mechanism comprises a breathable venting cord.

22. The needle assembly of Claim 18, wherein said venting mechanism comprises a textured surface.

23. The needle assembly of Claim 1, wherein said multiple sample sleeve comprises said venting mechanism

24. The needle assembly of Claim 23, wherein said venting mechanism comprises a vent media.

25. The needle assembly of Claim 23, wherein said multiple sample sleeve is formed from a porous hydrophobic material or said multiple sample sleeve comprises a porous material with a hydrophobic surface.

26. The needle assembly of Claim 1, wherein at least a portion of said hub is formed from a vent media such that said hub constitutes said venting mechanism.

27. The needle assembly of Claim 1, wherein at least a portion of said hub is formed from a porous material such that said hub constitutes said venting mechanism.

28. The needle assembly of Claim 1, wherein said hub further comprises a non-patient thread assembly and a hub insert, which form a reservoir when assembled together.

29. The needle assembly of Claim 28, wherein said venting mechanism comprises a first channel through said non patient thread assembly to said reservoir, a second channel from said reservoir to said ambient surroundings, and a venting plug located in said reservoir.

30. The needle assembly of Claim 1, further comprising a non-patient thread assembly, wherein at least a portion of said non-patient thread assembly is formed from a vent media such that said non-patient thread assembly constitutes said venting mechanism.

31. The needle assembly of Claim 1, further comprising a non-patient thread assembly, wherein at least a portion of said non-patient thread assembly is formed from a porous material such that said non-patient thread assembly constitutes said venting mechanism.

32. The needle assembly of Claim 1, wherein said venting mechanism comprises a vent media.

33. The needle assembly of Claim 1, further comprising a shield for selectively covering said first cannula.

34. A needle assembly comprising:
a hub having an inlet end, an outlet end and a chamber formed between said ends;
a cannula having opposite distal and proximal ends and a lumen extending between said ends, said cannula being mounted to said hub such that said distal end of said cannula is external of said hub;
a multiple sample sleeve mounted over portions of said proximal end of said cannula disposed externally of said hub, wherein said lumen, the internal surface of said multiple sample sleeve and said chamber, form a fluid passage; and
a venting mechanism providing communication between said fluid passage and ambient surroundings, wherein said venting mechanism permits an outflow of air from the needle assembly to said ambient surroundings through said venting mechanism, and wherein said venting mechanism substantially prevents an outflow of fluid from said needle assembly to said ambient surroundings through said venting mechanism.

35. The needle assembly of claim 34, wherein said venting mechanism is located in said passage beyond said proximal end of said outlet cannula

36. The needle assembly of claim 35, wherein said venting mechanism comprises a vent media

37. The needle assembly of: Claim 34, wherein said hub further comprises a non-patient barb and wherein said venting mechanism extends through said non-patient barb.

38. The needle assembly of claim 37, wherein said venting mechanism comprises a vent media.

39. The needle assembly of Claim 37, wherein said venting mechanism comprises a passage through said non patient barb and a venting plug located in said passage

40. The needle assembly of claim 37, wherein said venting mechanism comprises a slit through said non patient barb and a venting disc

41. The needle assembly of Claim 37, wherein said venting mechanism comprises at least one aperture through said non patient barb and a venting plug located in said aperture

42. The needle assembly of Claim 34, wherein said hub further comprises a non-patient barb, and wherein at least a portion of said non-patient barb comprises said venting mechanism

43. the needle assembly of claim 42, wherein said venting mechanism comprises a vent media.

44. The needle assembly of claim 42, wherein at least a portion of said non-patient barb is formed from a porous hydrophobic material or said portion of said non-patient barb comprises a porous material with a hydrophobic surface

45. The needle assembly of Claim 42, wherein said venting mechanism comprises a porous material impregnated with a hydrophilic material
